Europäisches Patentamt

**European Patent Office**

Office européen des brevets

(19)

(11) Publication number: **0 230 332**
**A1**

(12)

# EUROPEAN PATENT APPLICATION

(21) Application number: **87200031.0**

(51) Int. Cl.⁴: **A 61 K 9/22**

(22) Date of filing: **12.01.87**

(30) Priority: **13.01.86 NL 8600050**

(71) Applicant: **N.V. Sanico, Industrieterrein 48,
B-2300 Turnhout (BE)**
Applicant: **N.V. Gantax S.A., Brusselsestraat 96 B,
B-1681 Lennik (BE)**

(43) Date of publication of application: **29.07.87
Bulletin 87/31**

(72) Inventor: **Jansen, Frans HerwigJan, Witte Bremlaan 30,
B-2360 Oud Turnhout (BE)**
Inventor: **Hendrickx, Jean, Bogaardstraaat 25,
B-2300 Turnhout (BE)**

(84) Designated Contracting States: **AT BE CH DE ES FR GB
GR IT LI LU NL SE**

(74) Representative: **Kupecz, Arpad et al, Octrooibureau Los
en Stigter B.V. Postbox 20052, NL-1000 HB Amsterdam
(NL)**

(54) **A pharmaceutical composition with sustained release of the active component and a process for the preparation thereof.**

(57) The invention relates to a pharmaceutical composition, in particular in tablet form, with sustained release of the active component.

The pharmaceutical composition of the invention contains besides the active component a sugar ester of higher $C_{10}$-$C_{15}$ fatty acids in a suitable ratio to each other together with other suitable substances.

Usually, the sugar ester is a sucro ester and preferably sucrose monopalmitate.

Advantageously, the pharmaceutical composition of the invention besides the active component and the sugar ester furthermore contains a substance to control the release of the active component in the gastro-intestinal canal, such as PVP or a higher fatty acid ($C_{10}$-$C_{25}$), a higher alcohol ($C_{10}$-$C_{25}$) or fat (for instance stearinic acid).

Usually, the active component is a non-stereoidal anti-inflammatory compound and preferably Ibuprofen.

The invention also relates to a process for the preparation of the above pharmaceutical composition.

2x400 mg ibuprofen

A pharmaceutical composition with sustained release of the active component and a process for the preparation thereof.

The invention relates to a pharmaceutical composition, in particular in tablet form, with sustained release of the active component, and to a process for the preparation thereof.

In case of the most pharmaceutical compositions it is required that the plasm concentration of the product always remains above a minimum level in order to show an optimum therapeutical activity.

In general, it is easy to achieve, because when the pharmaceutical composition has been resorbed from the gastro-intestinal canal, the most products are secreted over an extended period of time. By using, for instance, compounds having a half-time of 15 to 20 hours, usually one tablet per day is sufficient. In case of products having a short half-time the above does not hold true, because too high peak concentrations should be avoided because of unsafety. This is for instance the case with Theophylline. In order to keep the concentration below a toxical level and to assure that the dose interval is sufficiently long, for instance 12 hours (two tablets per day) various ingenious systems are suggested: coatings, matrices, pellets with coatings, etc., for instance Theo-Dur, Theo 2, etc. In case of some products it is sufficient to use one tablet per day, for instance Voltaren Retard.

By means of various coatings, matrix systems, etc. it appeared to be possible to extend the dose interval for some pharmaceutical compositions, but in a way, which is clinically not satisfactory, because the patient still should take several tablets per day despite the retard phenomenon. Accordingly, it does not give any advantage for some pharmaceutical compositions with a short half-time compared with the single administration of a high dose of the pharmaceutical composition in the same dose interval.

The present invention now provides a pharmaceutical composition, in particular in tablet form, with sustained release of the active component, wherein the above-mentioned disadvantages are removed efficiently.

It is known that the use of sugar-fatty acid esters may result in improved pharmaceutical compositions such as an extended and quicker activity or improved tablet stability.

The pharmaceutical composition according to the invention is characterized in that the composition contains, besides the active compound, $C_{10}$-$C_{15}$ fatty acids in a suitable ratio to each other together with other suitable substances.

It is important that in case of a certain pharmacogenetic purpose the quantitative ratio of various ingredients is respected in order to obtain an optimum sustained release tablet.

It is surprising too that, when variations in the relative quantitative formulation do not result in differences in the in vitro tablets, the dissolution and desintegration graphics, the biological human effect may be different.

The pharmaceutical composition according to the invention preferably contains besides the active component and the sugar ester a substance for controlling the release of the active component in the gastro-intestinal canal.

Such a substance to control the release of the active component is for instance polyvinyl pyrrolidone (PVP) or a higher fatty acid ($C_{10}$-$C_{25}$), a higher alcohol ($C_{10}$-$C_{25}$) or fat, for instance stearinic acid.

As examples of active components according to the invention may be mentioned non-steoridal anti-inflammatory compounds, furthermore, valproic acid and salts and derivatives thereof, theophylline, nicotinic acid, salts and derivatives thereof, and in general compounds with a short half-time of for instance up to 12 hours, reasons why usually such substances are to be used frequently. Furthermore, we have to do in this case with pharmaceutical compositions which are to be used continuously during an extended period of time.

In particular, the present invention relates to Ibuprofen as active component, since it appeared up to now not possible to obtain a tablet having the above-mentioned suitable release pattern by use of this valuable active compound. Another typical example of controlled release according to the above-mentioned invention is a sustained release form of 5-aza, wherein the active compound only may occur at the last twist of the small intestine.

It has surprisingly been found that when besides Ibuprofen as active compound, sucro ester sucrose monopalmitate (the monoester of sucrose esterified with fatty acid) is present in the proper ratio, a tablet may be obtained with the aimed release pattern.

Furthermore, it appeared advantageous when the pharmaceutical composition contains PVP (polyvinyl pyrrolidone) and stearinic acid, since these compounds may control the release process in the gastro-intestinal canal dependent on the intended purpose, which is very surprising.

Of course, the components which are present in the pharmaceutical composition should be pharmaceutically acceptable, whereas the released active component should be emulsifyable in the gastro-intestinal canal.

Usually, the pharmaceutical composition according to the invention contains 2 to 1200 mg of active compound per tablet and in case of Ibuprofen 2 to 800 mg per tablet.

Furthermore, the invention relates to a process for the preparation of the above-mentioned pharmaceutical composition.

The process according to the invention is characterized in that the active compound is mixed with $C_{10}-C_{15}$ fatty acids at a temperature of 15-65°C, followed by cooling of the obtained mixture and by granulating of the obtained mixture to a proper particle size, whereas the thus obtained mixture is mixed with sucro ester and other excipients and the obtained mixture is sieved and finally it is compressed to a tablet in a way which is known in itself.

If Ibuprofen is used as active component, preferably sucrose monopalmitate is used as sugar ester and as a

- 4 -

0230332

substance to control the release of the active component polyvinyl pyrrolidone or stearinic acid.

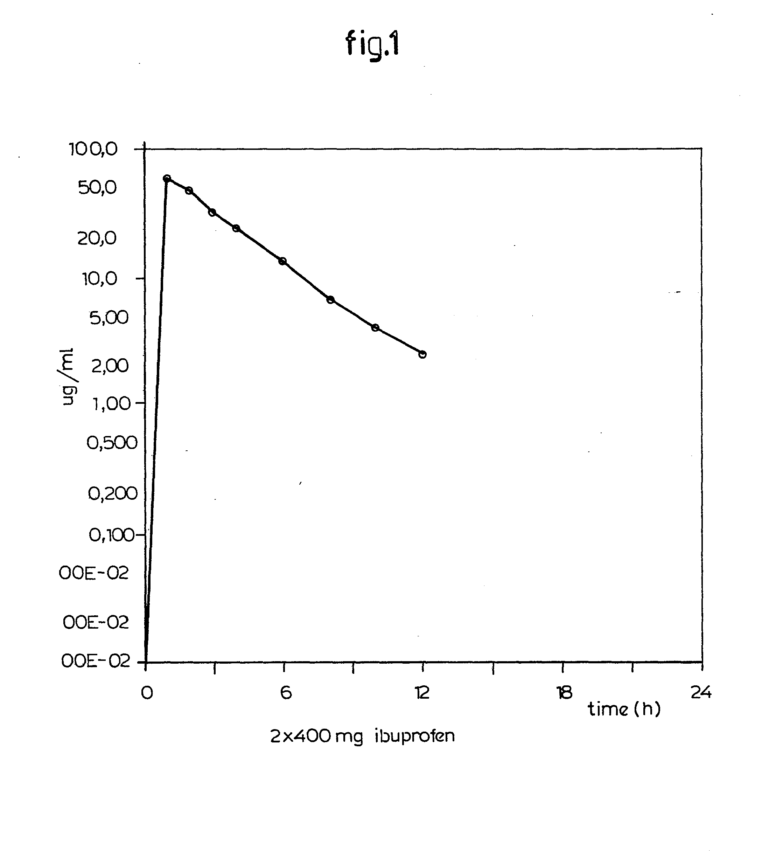

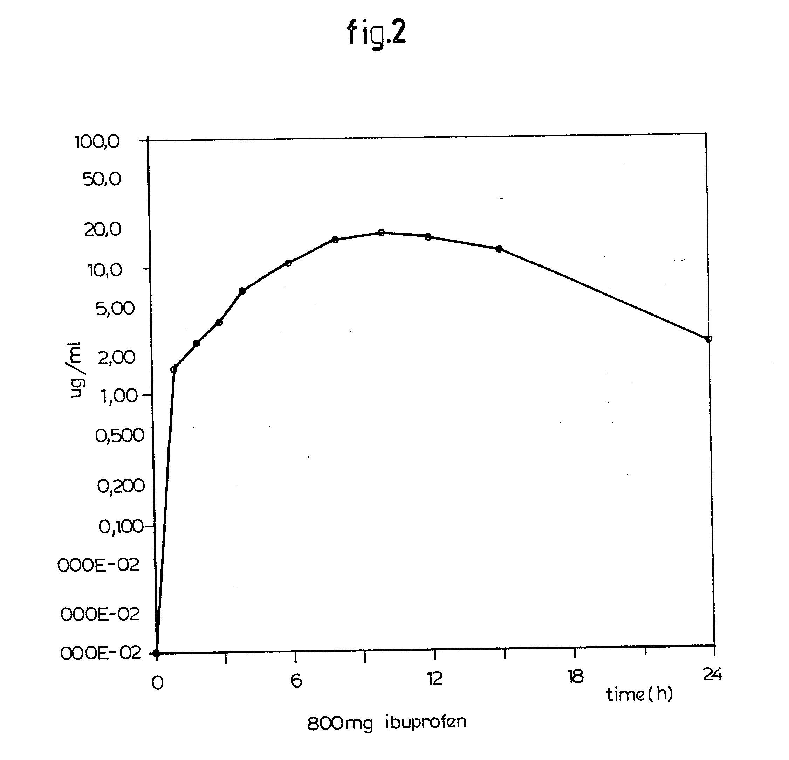

In case of Ibuprofen as active compound, the serum concentration of Ibuprofen is indicated in Figs. 1 and 2.

Fig. 1 shows the serum concentrations of Ibuprofen versus the time after oral administration of two tablets, each of them containing 400 mg of Ibuprofen, such as occurred prior to the invention.

Fig. 2 shows the serum concentrations of Ibuprofen versus the time after oral administration of one tablet which contains 800 mg Ibuprofen according to the invention.

Comparing Fig. 1 with Fig. 2, it clearly appears that the Ibuprofen containing usual tablets according to the invention show a significantly higher serum concentration of Ibuprofen than the known tablets.

According to Fig. 1 it appears that after 12 hours the serum concentration of Ibuprofen decreased from 50.0 µg/ ml to about 2.00 µg/ml, whereas according to Fig. 2 the serum concentration of Ibuprofen initially from 2.00 µg/ml gradual- ly increases to a maximum of about 18 µg and after 24 hours it achieves its final serum concentration of above 2.00 µg/ml.

Now the invention is illustrated by means of the following example, however, without any limitation of the invention thereto.

## EXAMPLE

Preparation of a 24-hours Ibuprofen containing tablet:

100 parts of Ibuprofen are mixed with 4 parts PVP and 10 parts of sucrose monopalmitate followed by granulation to the proper particle size. Then the product granules are mixed with 15 parts of sucrose monopalmitate and the usual excipients, such as dextrose, stearinic acid, talc and magnesium stearate. The obtained mixture was finally sieved. This mixture was then compressed to tablets in a suitable form and at a suitable pressure.

0230332

For the preparation of 500,000 tablets the following substances were required:

| | | |
|---|---:|---|
| Ibuprofen | 400 | kg |
| Dextrose monohydrate | 60 | kg |
| Polyvidon | 18 | kg |
| Sucrose monopalmitate | 100 | kg |
| Stearinic acid | 1 | kg |
| Talc | 16 | kg |
| Magnesium stearate | 5 | kg. |

CLAIMS

1.      A pharmaceutical composition, in particular in tablet form, with sustained release of the active component, c h a r a c t e r i z e d  in that besides the active component the composition contains a sugar ester of higher $C_{10}-C_{15}$ fatty acids in a suitable ratio to each other together with other suitable substances.

2.      The pharmaceutical composition according to claim 1, c h a r a c t e r i z e d  in that the sugar ester is a sucro ester.

3.      The pharmaceutical composition according to claim 2, c h a r a c t e r i z e d  in that the sucro ester is sucrose monopalmitate.

4.      The pharmaceutical composition according to claims 1-3, c h a r a c t e r i z e d  in that besides the active component and the sugar ester the composition contains a substance to control the release of the active component in the gastro-intestinal canal.

5.      The pharmaceutical composition according to claim 4, c h a r a c t e r i z e d  in that the substance to control the release of the active component is PVP or a higher fatty acid ($C_{10}-C_{25}$), a higher alcohol ($C_{10}-C_{25}$), or fat (for instance stearinic acid).

6.      The pharmaceutical composition according to claims 1-5, c h a r a c t e r i z e d  in that the active component is a non-stereoidal anti-inflammatory compound.

7.      The pharmaceutical composition according to claim 6, c h a r a c t e r i z e d  in that the active component is Ibuprofen.

8.      A process for the preparation of a pharmaceutical composition, in particular in tablet form, with sustained release of the active component, c h a r a c t e r i z e d  in that the active component is mixed with $C_{10}-C_{15}$ fatty acids at a temperature of 15-65°C, followed by cooling of the obtained mixture whereupon the obtained mass is granulated to a proper particle size, whereupon the obtained mixture is mixed with sucro ester and other excipients, then sieved and finally it is compressed to a tablet in a way which is known in itself.

- 7 -

0230332

9.      The process according to claim 8, c h a r a c -
t e r i z e d  in that as sugar ester sucro ester is used.
10        The process according to claim 9, c h a r a c -
t e r i z e d  in that as sucro ester sucrose monopalmitate
or the monoester of sucrose, esterified with a fatty acid, is
used.
11.      The process according to claims 8-10, c h a r a c -
t e r i z e d  in that a substance to control the release of
the active component in the gastro-intestinal canal is added
prior to the mixing of the active component with said fatty
acids.
12.      The process according to claim 11, c h a r a c -
t e r i z e d  in that the substance to control the release
of the active component is PVP or stearinic acid.
13.      The process according to claims 8-12, c h a r a c-
t e r i z e d  in that as active component Ibuprofen is used.

- 8 -

0230332

CLAIMS FOR AUSTRIA

1. A process for the preparation of a pharmaceutical composition, in particular in tablet form, with sustained release of the active component, c h a r a c t e r i z e d in that the active component is mixed with $C_{10}$-$C_{15}$ fatty acids at a temperature of 15-65°C, followed by cooling of the obtained mixture whereupon the obtained mass is granulated to a proper particle size, whereupon the obtained mixture is mixed with sucro ester and other excipients, then sieved and finally it is compressed to a tablet in a way which is known in itself.

2. The process according to claim 1, c h a r a c - t e r i z e d in that as sugar ester sucro ester is used.

3. The process according to claim 2, c h a r a c - t e r i z e d in that as sucro ester sucrose monopalmitate or the monoester of sucrose, esterified with a fatty acid, is used.

4. The process according to claims 1-3, c h a r a c- t e r i z e d in that a substance to control the release of the active component in the gastro-intestinal canal is added prior to the mixing of the active component with said fatty acids.

5. The process according to claim 4, c h a r a c - t e r i z e d in that the substance to control the release of the active component is PVP or a higher fatty acid ($C_{10}$-$C_{25}$), a higher alcohol ($C_{10}$-$C_{25}$) or fat (for instance stearinic acid).

6. The process according to claims 1-5, c h a r a c- t e r i z e d in that the active component is a non-steroidal anti-inflammatory compound.

7. The process according to claims 1-6, c h a r a c- t e r i z e d in that as active component Ibuprofen is used.

0230332

1/2

fig.1

2x400 mg ibuprofen

0230332

2/2

fig.2

800mg ibuprofen

# EUROPEAN SEARCH REPORT

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-1 080 265 (FARBENFABRIKEN BAYER AG) * Column 2, lines 30-35; claim * | 1-13 | A 61 K 9/22 |
| X | CHEMICAL ABSTRACTS, vol. 85, no. 6, 9th August 1976, page 282, abstract no. 37149m, Columbus, Ohio, US; S. JANICKI et al.: "Effect of sucrose monoesters on the physical properties of tablets containing meprobamate", & FARM. POL. 1976, 32(2), 113-116 | 1-3,8-11 | |
| X | CHEMICAL ABSTRACTS, vol. 75, no. 6, 9th August 1971, page 279, abstract no. 40363a, Columbus, Ohio, US; E. PAWLAK et al.: "Xanthinol nicotinate tablets with prolonged action. I. Moderation of the drug release by addition of various ancillary substances", & ACTA. POL. PHARM. 1971, 28(1), 43-47 | 1-5,8-13 | |
| X | CHEMICAL ABSTRACTS, vol. 73, no. 20, 16th November 1970, page 226, abstract no. 102065z, Columbus, Ohio, US; & CS-A-133 277 (L. BURDA et al.) 15-07-1969 | 1-13 | |

TECHNICAL FIELDS SEARCHED (Int. Cl.4)

A 61 K

-/-

The present search report has been drawn up for all claims

| Place of search | Date of completion of the search | Examiner |
|---|---|---|
| THE HAGUE | 16-04-1987 | BERTE M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

**EUROPEAN SEARCH REPORT**

| | DOCUMENTS CONSIDERED TO BE RELEVANT | | Page 2 |

| Category | Citation of document with indication, where appropriate, of relevant passages | Relevant to claim | CLASSIFICATION OF THE APPLICATION (Int. Cl.4) |
|---|---|---|---|
| X | WO-A-8 302 230 (F. DIETLIN)<br><br>* Page 3, lines 15-28; claims * | 1-6,8-12 | |
| | ----- | | |

TECHNICAL FIELDS SEARCHED (Int. Cl.4)

The present search report has been drawn up for all claims

| Place of search | Date of completion of the search | Examiner |
|---|---|---|
| THE HAGUE | 16-04-1987 | BERTE M.J. |